# EUROPÄISCHE PATENTSCHRIFT

(11) **EP 0 998 920 B1**
(45) Veröffentlichungstag und Bekanntmachung des Hinweises auf die Patenterteilung: **19.07.2006**
(21) Anmeldenummer: 99120464.5
(22) Anmeldetag: 14.10.1999
(51) Int. Cl.: A61K 9/20

(54) **Verfahren zur Herstellung von festen Dosierungsformen**
Method for producing solid dosage forms
Procede de production de formes solides de dosage

(30) Priorität: 15.10.1998 DE 19847618
(43) Veröffentlichungstag der Anmeldung: 10.05.2000
(73) Patentinhaber: Abbott GmbH & Co. KG, 65205 Wiesbaden (DE)
(72) Erfinder: Kothrade, Stephan, 67117 Limburgerhof (DE); Breitenbach, Jörg, 68199 Mannheim (DE); Krull, Harald, 67071 Ludwigshafen (DE); Kessler, Thomas, 67105 Schifferstadt (DE); Lange, Armin, 69121 Heidelberg (DE); Maier, Werner, 67105 Schifferstadt (DE); Reinhold, Ulrich, 67346 Speyer (DE)
(74) Vertreter: Kinzebach, Werner

(56) Entgegenhaltungen:
- DE-A- 19 531 684
- DE-A- 19 812 688
- US-A- 5 073 379

## Beschreibung

Die Erfindung betrifft ein Verfahren zur Herstellung von festen Dosierungsformen, insbesondere von festen pharmazeutischen Dosierungsformen.

Seit einiger Zeit ist ein kontinuierliches Verfahren zur Herstellung fester pharmazeutischer Formen bekannt, bei dem man eine wirkstoffhaltige, lösungsmittelfreie Schmelze aus einem polymeren, wirkstoffhaltigen Bindemittel extrudiert und den extrudierten Strang zu der gewünschten Arzneiform formt, beispielsweise in einem Kalander mit Formwalzen, siehe EP-A-240 904, EP-A-240 906, EP-A-337 256 und EP-A-358 105 (Schmelzextrusion). Damit kann eine gezielte Formgebung erreicht werden. Als polymeres Bindemittel werden insbesondere Polymere des N-Vinylpyrrolidon oder Copolymerisate davon, z. B. mit Vinylacetat, eingesetzt.

Das bekannte Verfahren weist den Nachteil auf, dass polymere Bindemittel mit einem K-Wert nach Fikentscher von mehr als 75, insbesondere Homo- oder Copolymere von Vinylpyrrolidon, nicht verarbeitet werden können, da sie bei den erforderlichen Temperaturen und/oder Verweilzeiten im Extruder Vernetzungen, Verfärbungen oder Zersetzung zeigen. In der pharmazeutischen Technologie gängige Hilfsstoffe, wie z. B. Polyvinylpyrrolidon mit einem K-Wert von 90, konnten daher bislang zur Herstellung von Dosierungsformen durch Extrusion nicht verwendet werden. Die Verwendung polymerer Bindemittel mit hohen K-Werten ist interessant zur Herstellung von sogenannten festen Lösungen, die eine Retardierung der Wirkstofffreisetzung gestatten.

Der vorliegenden Erfindung liegt daher die Aufgabe zugrunde, ein Verfahren zur Herstellung von festen Dosierungsformen durch Formung eines plastischen Gemisches bereitzustellen, das den Einsatz von hochmolekularen polymeren Bindemitteln zuläßt.

Überraschenderweise wurde gefunden, dass diese Aufgabe gelöst wird, wenn bei dem Verfahren die Temperatur und der Scherenergieeintrag kontrolliert werden.

Gegenstand der vorliegenden Erfindung ist daher ein Verfahren zur Herstellung von festen Dosierungsformen, bei dem man (i) ein plastisches Gemisch aus mindestens einem pharmakologisch akzeptablen polymeren Bindemittel mit einem K-Wert nach Fikentscher von mehr als 75, mindestens einem pharmazeutischen Wirkstoff und gegebenenfalls üblichen pharmazeutischen Additiven herstellt und (ii) das plastische Gemisch zur gewünschten Dosierungsform formt, wobei man den Schritt (i) in einem kontinuierlich arbeitenden Kneter und unter solchen Bedingungen der Temperatur und des Scherenergieeintrags vornimmt, dass der Molekulargewichtsabbau des polymeren Bindemittels, ausgedrückt als Differenz des K-Werts, weniger als 15, vorzugsweise weniger als 10, beträgt.

Unter Dosierungsformen sind hier alle Formen zu verstehen, die zur Verwendung als Arzneimittel, Pflanzenbehandlungsmittel, Futtermittel und Nahrungsmittel und zur Abgabe von Riechstoffen und Parfümölen geeignet sind. Dazu gehören beispielsweise Tabletten jeglicher Form, Pellets, Granulate, aber auch größere Formen, wie Würfel, Blöcke (Quader) oder zylindrische Formen, die sich insbesondere als Futter- oder Nahrungsmittel verwenden lassen.

Die erfindungsgemäß erhältlichen Dosierungsformen umfassen im Allgemeinen:
a) 0,1 bis 90 Gew.-%, insbesondere 0,1 bis 60 Gew.-% (bezogen
auf das Gesamtgewicht der Dosierungsform) eines Wirkstoffes, b) 10 bis 99,9 Gew.-%, insbesondere 40 bis 99,9 Gew.-% eines polymeren Bindemittels und
c) gegebenenfalls Additive.

Das polymere Bindemittel besitzt einen K-Wert von mehr als 75, insbesondere mehr als 80, vorzugsweise mehr als 85 und besonders bevorzugt 90-200. Die K-Werte werden nach H. Fikentscher, Cellulose-Chemie, Band 13, (1932) 58-64 und 71-74, in wässriger Lösung oder in einem organischen Lösungsmittel bei 25°C, bei Konzentrationen bestimmt, die je nach K-Wert-Bereich zwischen 0,1% und 5% liegen. Der K-Wert von wasserlöslichen Polymeren wird im Allgemeinen in wässriger Lösung bestimmt. Bei nicht vollständiger Löslichkeit des Polymeren in Wasser werden Lösungsmittel, wie THF, Aceton, Alkohole, z.B. Ethanol, eingesetzt.

Der Schritt (i) des erfindungsgemäßen Verfahrens wird unter solchen Bedingungen der Temperatur und des Scherenergieeintrags vorgenommen, dass der Molekulargewichtsabbau des polymeren Bindemittels, ausgedrückt als Differenz des K-Wertes, weniger als 15, vorzugsweise weniger als 10, beträgt. Geeignete Verfahrensparameter kann der Fachmann anhand einfacher Versuche leicht ermitteln. Hierzu wird der K-Wert des polymeren Bindemittels in der erhaltenen Dosierungsform bestimmt und mit dem K-Wert des eingesetzten polymeren Bindemittels verglichen. Die Verfahrensparameter können so variiert werden, dass bei ausreichender Vermischung der Molekulargewichtsabbau des polymeren Bindemittels so gering wie möglich ist.

Als Bindemittel geeignet sind Polymere, Copolymere, Cellulosederivate und Stärkederivate, beispielsweise:
Polyvinylpyrrolidon (PVP), Copolymerisate von N-Vinylpyrrolidon (NVP) und Vinylacetat oder Vinylpropionat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate (Eudragit-Typen), Copolymerisate von Methylmethacrylat und Acrylsäure, Polyacrylamide, Polyethylenglykole, Polyvinylformamid (gegebenenfalls partiell oder vollständig hydrolysiert), Celluloseester, Celluloseether, insbesondere Methylcellulose und Ethylcellulose, Hydroxyalkylcellulosen, insbesondere Hydroxypropylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane. Davon sind Polyvinylpyrrolidon, Copolymerisate von N-Vinylpyrrolidon und vinylestern, Polyhydroxyalkylacrylate, Polyhydroxyalkylmethacrylate, Polyacrylate, Polymethacrylate, Alkylcellulosen und Hydroxyalkylcellulosen besonders bevorzugt.

Homo- oder Copolymere von Vinylpyrrolidon, insbesondere solche mit mindestens 1 Gew.-%, vorzugsweise mindestens 10 Gew.-%, besonders bevorzugt mindestens 25 Gew.-% und insbesondere mindestens 50 Gew.-% Vinylpyrrolidoneinheiten, sind bevorzugt. Geeignete Comonomeren sind Vinylester von aliphatischen C₂-C₂₄-Carbonsäuren, wie Vinylacetat oder Vinylpropionat; C₁-C₂₄-Alkyl(meth)acrylate, wie Methylmethacrylat, Ethylacrylat, Stearyl(meth)acrylat; Vinylether, wie Methylvinylether. Hydrophobe Comonomere sind im Allgemeinen bevorzugt.

Das polymere Bindemittel muß in der Gesamtmischung aller Komponenten im Bereich von 70 bis 300 °C, vorzugsweise 80 bis 250 °C erweichen, wobei sich ein plastisches Gemisch bildet. Die Glasübergangstemperatur der Mischung muss daher unter 250 °C, vorzugsweise unter 200 °C liegen.

In bevorzugten Ausführungsformen wird die Glasübergangstemperatur durch Zusatz pharmakologisch akzeptabler weichmachender Hilfsstoffe herabgesetzt. Die Menge an Weichmacher beträgt im Allgemeinen 0,5 bis 30, vorzugsweise 0,5 bis 15 Gew.-%, bezogen auf das Gesamtgewicht des Gemisches.

Beispiele für derartige Weichmacher sind:
langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylenpropylenglykole, Silicone, aromatische Carbonsäureester (z. B. Dialkylphthalate, Trimellithsäureester, Benzoesäureester, Terephthalsäureester) oder aliphatische Dicarbonsäureester (z. B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat, wovon Polyethylenglycole und Polyethylenpropylenglycole bevorzugt sind.

Die Verwendung eines Weichmachers führt dazu, dass die Erweichungstemperatur des polymeren Bindemittels sinkt. Die Bildung des plastischen Gemisches und die Formgebung können bei niedrigeren Temperaturen erfolgen, wodurch der Molekulargewichtsabbau eingeschränkt wird.

In bevorzugten Ausführungsformen enthält das plastische Gemisch ferner pharmazeutisch akzeptable Antioxidantien. Diese können in einer Menge von 0,001 bis 10 Gew.-%, vorzugsweise 0,01 bis 5 Gew.-%, bezogen auf das Gesamtgewicht des Gemisches, verwendet werden. Geeignete Antioxidantien sind z. B. Gallussäureester, Ascorbylpalmitat, Ascorbinsäure, Butylhydroxytoluol, Butylhydroxyanisol, Tocopherole, Nordihydroguajaretsäure, 2,6-Di-tert-butyl-4-methylphenol, Alkali- oder Erdalkalisulfite oder -bisulfite und Gemische davon.

Durch Mitverwendung von Antioxidantien kann der Molekulargewichtsabbau des polymeren Bindemittels beim erfindungsgemäßen Verfahren weiter eingeschränkt werden.

Übliche galenische Hilfsstoffe, deren Gesamtmenge bis zu 100 Gew.-%, bezogen auf das Polymerisat, betragen kann, sind z. B. Streckmittel bzw. Füllstoffe, wie Silikate oder Kieselerde, Magnesiumoxid, Aluminiumoxid, Titanoxid, Stearinsäure oder deren Salze, z. B. das Magnesium- oder Calciumsalz, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches;

Schmiermittel, wie Aluminium-, Magnesium- und Calciumstearat, Talkum und Silicone, in einer Konzentration von 0,1 bis 5, vorzugsweise 0,1 bis 3 Gew.-%, bezogen auf das Gesamtgewicht des Gemisches;
Fließmittel, wie tierische oder pflanzliche Fette, insbesondere in hydrierter Form und solche, die bei Raumtemperatur fest sind. Diese Fette haben vorzugsweise einen Schmelzpunkt von 50°C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Fetten, Wachsen, Mono-, Diglyceriden und/oder Lecithinen beträgt 0,1 bis 30, vorzugsweise 0,1 bis 5 Gew.-%, bezogen auf das Gesamtgewicht der Masse für die jeweilige Schicht;

Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;

Stabilisatoren, wie Lichtstabilisatoren, Hydroperoxid-Vernichter, Radikalfänger, Stabilisatoren gegen mikrobiellen Befall.

Ferner können Netz-, Konservierungs-, Spreng-, Adsorptions-, Formentrenn- und Treibmittel zugesetzt werden (vgl. z. B. H. Sucker et al., Pharmazeutische Technologie, Thieme-Verlag, Stuttgart 1978).

Unter Hilfsstoffen im Sinne der Erfindung sind auch Substanzen zur Herstellung einer festen Lösung des Wirkstoffs zu verstehen. Diese Hilfsstoffe sind beispielsweise Pentaerythrit und Pentaerythrit-tetraacetat, Polymere wie z. B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere, wie z. B. bei J. L. Ford, Pharm. Acta Helv. 61, 69-88 (1986) angegeben.

Als Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Einzige Voraussetzung für die Eignung von Hilfsstoffen ist eine ausreichende Temperaturstabilität.

Unter Wirkstoffen im Sinne der Erfindung sind alle Stoffe mit einer physiologischen Wirkung zu verstehen, sofern sie sich unter den Verarbeitungsbedingungen nicht zersetzen. Es handelt sich insbesondere um pharmazeutische Wirkstoffe (für Mensch und Tier), Wirkstoffe für die Pflanzenbehandlung, Insektizide, Futter- und Nahrungsmittelwirkstoffe, Riechstoffe und Parfümöle. Die Wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.-% liegen. Auch Wirkstoff-Kombinationen können eingesetzt werden. Wirkstoffe im Sinne der Erfindung sind auch Vitamine und Mineralstoffe. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika. Zu Pflanzenbehandlungsmitteln zählen z. B. Vinclozolin, Epoxiconazol und Quinmerac.

Das erfindungsgemäße Verfahren ist beispielsweise zur Verarbeitung folgender Wirkstoffe geeignet:
Acebutolol, Acetylcystein, Acetylsalicylsäure, Aciclovir, Alprazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefadroxil, Cefazolin, Cefixim, Cefotaxim, Ceftazidim, Ceftriaxon, Cefuroxim, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Folinsäure, Furosemid, Gallopamil, Gemfibrozil, Gentamicin, Gingko biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Imipramin, Indomethacin, Iohexol, Iopamidol, Isosorbid-Dinitrat, Isosorbid-Mononitrat, Isotretinoin, Itraconazol, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Pentoxifyllin, Phenobarbital, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Selegilin, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Zidovudin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin oder Captopril.

Zur Herstellung der festen Dosierungsformen wird ein plastisches Gemisch der Komponenten bereitgestellt, das anschließend einem Formgebungsschnitt unterzogen wird. Das Vermischen der Komponenten und die Bildung des plastischen Gemisches können auf unterschiedliche Weise erfolgen. Das Vermischen kann vor, während und/oder nach der Bildung des plastischen Zustands erfolgen. Beispielsweise können die Komponenten zuerst vermischt und dann erweicht oder gleichzeitig vermischt und erweicht werden. Wenn eine Homogenisierung des plastischen Gemisches erfolgt, um eine hochdisperse Verteilung des Wirkstoffes zu erhalten, so muß diese unter niedrigscherenden Bedingungen durchgeführt werden.

Insbesondere bei Verwendung von empfindlichen Wirkstoffen hat es sich aber als bevorzugt erwiesen, zuerst das polymere Bindemittel, gegebenenfalls zusammen mit üblichen pharmazeutischen Additiven, zu erweichen und vorzuvermischen und dann den (die) empfindlichen Wirkstoff(e) in "Intensivmischern" in plastischer Phase bei sehr kleinen Verweilzeiten einzumischen (Homogenisieren). Der (die) Wirkstoff(e) kann (können) dabei in fester Form oder als Lösung oder Dispersion eingesetzt werden.

Im Allgemeinen werden die Komponenten als solche in das Herstellungsverfahren eingesetzt. Sie können jedoch auch in flüssiger Form, d. h. als Lösung, Suspension oder Dispersion zur Anwendung kommen.

Als Lösungsmittel für die flüssige Form der Komponenten kommt in erster Linie Wasser oder ein mit Wasser mischbares, organisches Lösungsmittel oder ein Gemisch davon mit Wasser in Betracht. Brauchbare Lösungsmittel sind aber auch mit Wasser nicht mischbare organische Lösungsmittel. Geeignete, mit Wasser mischbare Lösungsmittel sind insbesondere C₁-C₄-Alkanole, wie Ethanol, Isopropanol oder n-Propanol, Polyole, wie Ethylenglykol, Glycerin und Polyethylenglykole. Geeignete, mit Wasser nicht mischbare Lösungsmittel sind Alkane, wie Pentan oder Hexan, Ester, wie Ethylacetat oder Butylacetat, chlorierte Kohlenwasserstoffe, wie Methylenchlorid und aromatische Kohlenwasserstoffe, wie Toluol und Xylol. Ein weiteres brauchbares Lösungsmittel ist flüssiges CO₂.

Welches Lösungsmittel im Einzelfall verwendet wird, hängt von der aufzunehmenden Komponente und deren Eigenschaften ab. Beispielsweise kommen pharmazeutische Wirkstoffe häufig in Form eines Salzes, das im Allgemeinen wasserlöslich ist, zur Anwendung. Wasserlösliche Wirkstoffe können daher als wässrige Lösung eingesetzt werden oder vorzugsweise in die wässrige Lösung oder Dispersion des Bindemittels aufgenommen werden. Entsprechendes gilt für Wirkstoffe, die in einem der genannten Lösungsmittel löslich sind, wenn die flüssige Form der zur Anwendung kommenden Komponenten auf einem organischen Lösungsmittel basiert.

Gegebenenfalls kann an die Stelle des Erweichens ein Lösen, Suspendieren oder Dispergieren in den oben genannten Lösungsmitteln, falls erwünscht und/oder erforderlich unter Zusatz geeigneter Hilfsstoffe, wie z. B. Emulgatoren, treten. Das Lösungsmittel wird dann im Allgemeinen unter Bildung des plastischen Gemischesin einer geeigneten Apparatur, z. B. einer Knet- und Plastiziervorrichtung, entfernt. Im Folgenden soll dies von dem Begriff Vermischen umfasst werden.

Das Erweichen und Vermischen erfolgt unter solchen Bedingungen der Temperatur und des Scherenergieeintrags, dass der Molekulargewichtsabbau des polymeren Bindemittels, ausgedrückt als Differenz des K-Werts, weniger als 15 beträgt. Die zu diesem Zweck verwendete Vorrichtung muß unter diesem Gesichtspunkt ausgewählt werden. Üblicherweise zur Schmelzextrusion herangezogene Doppelwellenschneckenextruder sind in der Regel nicht geeignet. Die Kopplung des Aufschmelz- und Mischvorgangs im Extruder erfordert, um eine ausreichende Vermischung zu bewirken, eine relativ lange Verweilzeit in einer Zone mit hoher Scherung. Dadurch kann es zu einer lokalen Überhitzung und einem übermäßigen Molekulargewichtsabbau des Bindemittels kommen. Weiterhin ist zu bedenken, dass zur Vermeidung einer Schädigung des polymeren Bindemittels die Temperatur nicht beliebig gesteigert werden kann. Bei vergleichsweise niedrigen Temperaturen zeigen die erfindungsgemäß eingesetzten polymeren Bindemittel mit einem K-Wert von mehr als 80 jedoch eine sehr hohe Viskosität. Doppelwellenextruder können unter diesen Bedingungen zu Blockieren neigen.

Das erfindungsgemäße Verfahren muß in einem kontinuierlich arbeitendem Kneter durchgeführt werden. Bevorzugte Kneter weisen eine mit einer Schneckenwendel versehene Welle in einem zylindrischen Gehäuse auf, wobei die Welle zusätzlich zur Rotationsbewegung um ihre eigene Achse eine axiale Hin- und Herbewegung ausführt. Vorzugsweise ist die Schneckenwendel mehrfach unterbrochen und weist das Gehäuse feststehende Knetzähne auf, wobei die Knetzähne bei der Hin- und Herbewegung der Welle durch die Unterbrechungen hindurchgehen. Derartige kontinuierlich arbeitende Kneter werden unter der Bezeichnung Ko-Kneter von der Firma Buss angeboten.

Das Beschicken der Misch- und Plastifiziervorrichtung erfolgt je nach deren Konzeption kontinuierlich oder diskontinuierlich in üblicher Weise. Pulverförmige Komponenten können im freien Zulauf, z. B. über eine Differentialdosierwaage eingeführt werden. Plastische Massen können direkt oder über eine Zahnradpumpe, die insbesondere bei hohen Viskositäten und hohen Drücken von Vorteil ist, zugespeist werden. Flüssige Medien können über ein geeignetes Pumpenaggregat zudosiert werden.

Das durch Vermischen und/oder Erweichen des Bindemittels, des Wirkstoffes und gegebenenfalls des Additivs oder der Additive erhaltene Gemisch ist meist teigig bis zähflüssig (thermoplastisch). Die Glasübergangstemperatur des Gemisches liegt unter der Zersetzungstemperatur aller in dem Gemisch enthaltenen Komponenten. Das Bindemittel soll vorzugsweise in physiologischer Umgebung löslich oder quellbar sein.

Die Verfahrensschritte Vermischen und Erweichen können in derselben Apparatur oder in zwei oder mehreren getrennt arbeitenden Vorrichtungen ausgeführt werden. Die Zubereitung einer Vormischung kann in einer der oben beschriebenen üblichen Mischvorrichtungen durchgeführt werden. Eine solche Vormischung kann dann direkt, z. B. in einen kontinuierlichen Kneter, eingespeist und anschließend ggf. unter Zusatz weiterer Komponenten extrudiert werden.

Erfindungsgemäß können auch mehrschichtige pharmazeutische Formen durch Koextrusion hergestellt werden, wobei mehrere Gemische aus den oben beschriebenen Komponenten bei der Extrusion so in einem Werkzeug zusammengeführt werden, dass sich der gewünschte Schichtaufbau der mehrschichtigen pharmazeutischen Form ergibt. Vorzugsweise verwendet man verschiedene Bindemittel für verschiedene Schichten, wobei in einer Schicht ein Bindemittel mit einem K-Wert von mehr als 75 eingesetzt wird.

Mehrschichtige Arzneiformen umfassen vorzugsweise zwei oder drei Schichten. Sie können in offener oder geschlossener Form vorliegen, insbesondere als offene oder geschlossene Mehrschichttabletten.

Wenigstens eine der Schichten enthält wenigstens einen pharmazeutischen Wirkstoff. Es ist auch möglich, einen weiteren Wirkstoff in eine andere Schicht aufzunehmen. Dies hat den Vorteil, dass zwei miteinander unverträgliche Wirkstoffe verarbeitet werden können oder dass die Freisetzungscharakteristik des Wirkstoffes gesteuert werden kann.

Das Ausformen erfolgt durch Koextrusion, wobei die Gemische aus den einzelnen Extrudern oder anderen Aggregaten in ein gemeinsames Koextrusionswerkzeug geführt und ausgetragen werden. Die Form der Koextrusionswerkzeuge richtet sich nach der gewünschten pharmazeutischen Form. Beispielsweise sind Werkzeuge mit ebenem Austrittsspalt, sogenannte Breitschlitzwerkzeuge, und Werkzeuge mit kreisringspaltförmigem Austrittsquerschnitt geeignet. Die Düsenauslegung erfolgt dabei in Abhängigkeit von dem zur Anwendung kommenden polymeren Bindemittel und der gewünschten pharmazeutischen Form.

Das erhaltene Gemisch ist vorzugsweise lösungsmittelfrei, d.h. es enthält weder Wasser noch ein organisches Lösungsmittel.

Das plastische Gemisch wird in der Regel einer abschließenden Formgebung unterzogen. Dabei kann eine Vielzahl von Formen, je nach Werkzeug und Art der Formung, erzeugt werden. Beispielsweise lässt sich bei Verwendung eines Extruders der extrudierte Strang zwischen einem Band und einer Walze, zwischen zwei Bändern oder zwischen zwei Walzen, wie in der EP-A-358 105 beschrieben, oder durch Kalandrierung in einem Kalander mit zwei Formwalzen, siehe beispielsweise EP-A-240 904, formen. Durch Extrusion und Heiß-oder Kaltabschlag des Stranges können weitere Formen erhalten werden, beispielsweise kleinteilige und gleichmäßig geformte Granulate. Die Heißgranulierung führt in der Regel zu linsenförmigen Dosierungsformen (Tabletten) mit einem Durchmesser von 1 bis 10 mm, während die Kaltgranulierung normalerweise zu zylinderförmigen Produkten mit einem Verhältnis von Länge zu Durchmesser von 1 bis 10 und einem Durchmesser von 0,5 bis 10 mm führt. So können einschichtige, bei Anwendung der Koextrusion aber auch offene oder geschlossene, mehrschichtige Dosierungsformen hergestellt werden, beispielsweise Oblongtabletten, Dragees, Pastillen und Pellets. Die erhaltenen Granulate können anschließend auch zu Pulver gemahlen und in üblicher Weise zu Tabletten verpresst werden. Mikropastillen können durch das Rotoform-Sandvik-Verfahren hergestellt werden. Diese Dosierungsformen können in einem nachgeschalteten Verfahrensschritt nach üblichen Methoden gerundet und/oder mit einem Coating versehen werden. Geeignete Materialien für Filmüberzüge sind z. B. Polyacrylate, wie die Eudragit-Typen, Celluloseester, wie die Hydroxypropylcellulosephthalate, sowie Celluloseether, wie Ethylcellulose, Hydroxypropylmethylcellulose oder Hydroxypropylcellulose.

Im Einzelnen kann es zur Ausbildung von festen Lösungen kommen. Der Begriff "feste Lösungen" ist dem Fachmann geläufig, beispielsweise aus der eingangs zitierten Literatur. In festen Lösungen von Wirkstoffen in Polymeren liegt der Wirkstoff molekulardispers im Polymer vor.

Die folgenden Beispiele sollen das erfindungsgemäße Verfahren veranschaulichen, ohne es jedoch zu beschränken.

### Beispiele

### Beispiel 1

Polyvinylpyrrolidon (Pulver) mit einem K-Wert von 90, dem 20 Gew.-% Ibuprofen beigemischt war, wurde auf einem Ko-Kneter BussMDK 46 der Firma Buss AG, Schweiz, extrudiert. Die verwendete Welle wies Vorschubabschnitte mit durchgängiger Schneckenwendel und Knetabschnitte mit unterbrochener Schneckenwendel und feststehenden Knetzähnen im Wechsel auf. Die Einspeisung des Polyvinylpyrrolidons erfolgte im letzten Drittel der Welle. Die Temperatur wurde so gesteuert, dass sie von 80°C an der Eintrittsstelle auf 195°C an der Austrittsstelle anstieg. Der Extruderstrang wurde mit Hilfe von Kühlwalzen und einem Kühlband rasch auf Raumtemperatur gebracht. Das Extrudat zeigte keine Verfärbung und wies einen K-Wert von 84,5 auf.

### Beispiel 2

Ein Gemisch von Polyvinylpyrrolidon mit einem K-Wert von 90, einem Gew.-% Stabilisator (Butylhydroxytoluol) und 20 Gew.-% Ibuprofen wurde wie in Beispiel 1 beschrieben extrudiert. Das Extrudat zeigte keine Verfärbung und wies einen K-Wert von 85,5 auf.

### Vergleichsbeispiel

Ein Gemisch von 85 Gew.-% Polyvinylpyrrolidon mit einem K-Wert von 90 und 15 Gew.-% Lutrol 1500 (Polyethylenglycol) wurde auf einem Doppelschneckenextruder ZSK 30 der Firma Werner und Pfleiderer extrudiert. Die Temperatur betrug 80 bis 205 °C. Das Extrudat zeigte eine braune Verfärbung und wies einen K-Wert von 60 auf.

## Patentansprüche

1. Verfahren zur Herstellung von festen Dosierungsformen, bei dem man
(i) ein plastisches Gemisch aus mindestens einem pharmakologisch akzeptablen polymeren Bindemittel mit einem K-Wert nach Fikentscher von mehr als 75, mindestens einem pharmazeutischen Wirkstoff und gegebenenfalls üblichen pharmazeutischen Additiven herstellt und
(ii) das plastische Gemisch zur gewünschten Dosierungsform formt,
wobei man den Schritt (i) in einem kontinuierlich arbeitenden Kneter und unter solchen Bedingungen der Temperatur und des Scherenergieeintrags vornimmt, dass der Molekulargewichtsabbau des polymeren Bindemittels, ausgedrückt als Differenz des K-Werts, weniger als 15 beträgt

2. Verfahren nach Anspruch 1, **dadurch gekennzeichnet, dass** das polymere Bindemittel ein Homo- oder Copolymer von Vinylpyrrolidon ist.

3. Verfahren nach Anspruch 1 oder 2, **dadurch gekennzeichnet, dass** die Temperatur bei dem Schritt (i) nicht mehr als 250 °C beträgt.

4. Verfahren nach einem der vorhergehenden Ansprüche, **dadurch gekennzeichnet, dass** der Kneter eine mit einer Schneckenwendel versehene Welle in einem zylindrischen Gehäuse aufweist, wobei die Welle zusätzlich zur Rotationsbewegung um ihre eigene Achse eine axiale Hin- und Herbewegung ausführt.

5. Verfahren nach Anspruch 4, **dadurch gekennzeichnet, dass** die Schneckenwendel mehrfach unterbrochen ist und das Gehäuse feststehende Knetzähne aufweist, wobei die Knetzähne bei der Hin- und Herbewegung der Welle durch die Unterbrechungen hindurch gehen.

6. Verfahren nach einem der vorhergehenden Ansprüche, **dadurch gekennzeichnet, dass** das plastische Gemisch wenigstens einen Weichmacher umfasst.

7. Verfahren nach einem der vorhergehenden Ansprüche, **dadurch gekennzeichnet, dass** das plastische Gemisch wenigstens ein Antioxidans umfasst.

## Claims

1. A process for producing solid dosage forms, in which
(i) a plastic mixture of at least one pharmacologically acceptable polymeric binder with a K value according to Fikentscher of more than 75, at least one pharmaceutical active ingredient and, if appropriate, conventional pharmaceutical additives is prepared and
(ii) the plastic mixture is shaped to the required dosage form,
with step (i) being carried out in a continuously operating kneader and under conditions of temperature and shear energy input such that the reduction in molecular weight of the polymeric binder, expressed as the difference in the K value, is less than 15.

2. The process according to claim 1, wherein the polymeric binder is a homo- or copolymer of vinylpyrrolidone.

3. The process according to claim 1 or 2, wherein the temperature in step (i) is not more than 250°C.

4. The process according to any of the preceding claims, wherein the kneader has a shaft provided with a screw flight in a cylindrical housing, and the shaft executes an axial movement to and fro in additional to the rotational movement around its own axis.

5. The process according to claim 4, wherein the screw flight has multiple interruptions, and the housing has fixed kneading cogs, with the kneading cogs passing through the interruptions during the movement to and fro of the shaft.

6. The process according to any of the preceding claims, wherein the plastic mixture comprises at least one plasticizer.

7. The process according to any of the preceding claims, wherein the plastic mixture comprises at least one antioxidant.

## Revendications

1. Procédé pour produire des formes galéniques solides dans lequel on
(i) produit un mélange plastique à partir d'au moins un liant polymère pharmacologiquement acceptable ayant une valeur K selon Fikentscher supérieure à 75, au moins un principe actif pharmaceutique et éventuellement des additifs pharmaceutiques courants et
(ii) met le mélange plastique sous forme de la forme galénique souhaitée,
où on réalise l'étape (i) dans un malaxeur fonctionnant en continu et dans des conditions de température et d'apport d'énergie de cisaillement telles que la dégradation de masse moléculaire du liant polymère, exprimée sous forme de différence de la valeur K, est inférieure à 15.

2. Procédé selon la revendication 1 **caractérisé en ce que** le liant polymère est un homo- ou copolymère de vinylpyrrolidone.

3. Procédé selon la revendication 1 ou 2 **caractérisé en ce que** la température dans l'étape (i) n'est pas supérieure à 250°C.

4. Procédé selon l'une des revendications précédentes **caractérisé en ce que** le malaxeur comporte un arbre muni d'une hélice de vis sans fin dans un boîtier cylindrique, où l'arbre décrit un mouvement de va-et-vient axial en plus du mouvement de rotation autour de son propre axe.

5. Procédé selon la revendication 4 **caractérisé en ce que** l'hélice de vis sans fin est interrompue plusieurs fois et le boîtier comporte des dents de malaxage fixes, où les dents de malaxage passent dans les interruptions lors du mouvement de va-et-vient de l'arbre.

6. Procédé selon l'une des revendications précédentes **caractérisé en ce que** le mélange plastique comprend au moins un plastifiant.

7. Procédé selon l'une des revendications précédentes **caractérisé en ce que** le mélange plastique comprend au moins un antioxydant.
